(19) Europäisches Patentamt
European Patent Office
Office européen des brevets

(11) **EP 3 933 850 A1**

(12) **EUROPEAN PATENT APPLICATION**

(43) Date of publication:
**05.01.2022  Bulletin 2022/01**

(51) Int Cl.:
**G16H 50/20** (2018.01)        **G16H 50/70** (2018.01)

(21) Application number: **20382572.4**

(22) Date of filing: **29.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koa Health B.V.**
**1097 JV, Amsterdam (NL)**

(72) Inventors:
• **Buda, Teodora Sandra**
  **28013 Madrid (ES)**

• **Matic, Aleksandar**
  **28013 Madrid (ES)**
• **Khwaja, Mohammed**
  **28013 Madrid (ES)**
• **Garriga Calleja, Roger**
  **28013 Madrid (ES)**
• **Estella, Iñaki**
  **28013 Madrid (ES)**

(74) Representative: **Carlos Hernando, Borja**
**Garrigues IP, S.L.P.**
**Hermosilla, 3**
**28001 Madrid (ES)**

(54) **METHOD, APPARATUS AND COMPUTER PROGRAMS FOR EARLY SYMPTOM DETECTION AND PREVENTATIVE HEALTHCARE**

(57)    A computer implemented method, an apparatus and computer programs for early symptom detection and preventative healthcare are provided. The method comprises accessing medical and/or wellness information gathered from a user; determining a risk predictive model using the accessed information; determining a risk of a specific health outcome using the risk predictive model; and defining a decision control mechanism that calibrates said risk predictive model by: computing a set of attribute scores defining freshness, sufficiency and reliability of accessed information; providing weights to the computed set of attribute scores; computing a first score relating to a need for more information of the user using said weights and the set of attribute scores; computing a second score relating to a need for a check-up of the user using different variables of interest and traits of the user; and determining that new information and/or a check-up is needed based on said first and second scores.

Fig. 1

**Description**

<u>Technical Field</u>

The present invention relates to a computer implemented method, an apparatus and computer programs for early symptom detection and preventative healthcare. The invention allows to dynamically setting data requirements and prompting for minimally required data to deliver early symptom detection and preventative healthcare by balancing between data requirements defined as completeness, freshness and uncertainty, such that for a specific healthcare outcome and for a specific patient it provides an accurate continuous risk prediction. The feedback loop between data availability, prediction confidence, data requirements, and the known outcome (ground-truth) enables individually tailored preventative care and early detection of health related symptoms with minimally required but sufficient data.

<u>Background of the Invention</u>

The Prior-Art in the domain of processing healthcare relevant data for enhancing therapy and interventions mainly covers the following broad areas: 1) data collection and its maintenance; 2) defining the treatment plan; 3) improving patients' adherence; and 4) raising alarms in critical moments.

Data collection: inventions in this domain focus mainly on how to aggregate and distribute medical information to the healthcare providers - such as in US 20110225007-A1 that focuses on delivering medical information to coaches. A more comprehensive way to handle personal health data is presented in US 7395215-B2 that ensures historical data consistency, completeness and availability when needed. In this case *completeness* refers to gathering all the data from historical records that can sit at different healthcare providers - in the present invention, *completeness* rather refers to inferring which data items are needed for a very specific purpose of early detection or prevention of symptoms. In that regard, the present invention does not count on all the available information, rather on the required information for the purpose and the data query if these needed data items do not exist.

Treatment: There are several inventions that define systems for monitoring mental health symptoms. The most relevant to the present invention is US 7540841-B2 that defines both monitoring mental health symptoms and creating a treatment plan. The treatment plan is defined according to the existing protocols. Inventions, understandably, focus on the existing protocols - such as CN 101526980-A which compares the applied therapy plan with the medical protocol. The present invention, in turn, goes beyond the existing protocols aiming to personalize the protocols not based on the diagnosis (which often can be wrong) but to each individual. Importantly, this does not imply the lack of compliance with the existing medical protocols, rather modifications of the protocol to each individual to maximize the therapy efficiency (note that effectiveness depends on the adherence) while optimizing healthcare staff engagement (e.g. for some patients the check-ups will be conducted with a lower and for some with a higher frequency whit reference to what is defined by the protocol).

Adherence: US 20130226617-A1 defines both the treatment plan and the ways to boost the adherence to it. The rating score combines individual rating scores for treatment steps of the plan into a single score. Similarly, US 8566121-B2 builds user profiles based on which the invention creates the interventions for minimizing medical non-adherence.

Alerts: This is a wide invention space, which spans from ensuring technical capabilities to deliver alerts when needed (i.e. system driven inventions) to defining the ways how to detect when the right time is to raise an alarm. For instance, US 20170098050-A1 and US 20060058612-A1 focus on the system for delivering health alarms and communication. US 5576952-A generates alerts based on monitoring units (mainly physiological parameters) and it provides a system and method for comparing against the selected limit parameters.

There are also inventions that are very specific to a certain condition - such as anger and stress - US 20080214944-A1 - which defines how to use health parameters for delivering the therapy. Similarly, US 20110245633-A1 tackles diagnosis, treatment and assessment of the treatment effects, using wearable devices.

The existing invention space covers the four mentioned areas, namely data gathering, treatment plans, adherence and alarms. With respect to these four domains, there is no an end to end system which:

- Goes beyond data gathering and maintenance to ensure the quality/certainty, completeness, freshness of the data required for a defined purpose. Rather, the inventions focus on general data completeness and gathering the available data collected either through Electronic Health Records or using new technologies.

- Treatment plans - are typically defined "in vacuum" i.e. with no regards to the prevention, and instead they are focused on treatment plans for the diagnosed conditions.

- Adherence - this space provides solutions for making patients adhere to the prescribed therapies. As much as this is important in the medical space, no invention defines when the adherence in data collection is needed i.e. when patients are critical to adhere to the check-ups (that result in gathering potentially predictive data of the future symptoms and to refining treatment plan with modified healthcare protocol).

- Alarms are typically based on detecting when specific parameters are outside of the accepted range and defining the ways to signalize this to the right caregiver/family member/healthcare staff

That is, there are no known end-to-end solutions that define a loop that enable an administration of the personalized healthcare protocol and the corresponding preventative care.

Description of the Invention

Providing the right intervention at the right time based on continuously acquired information about a user lies at the core of present invention's strategy and its competitive advantage. Preventative services represent the holy grail of healthcare - for healthy individuals (to prevent negative health outcomes in the future) and for already diagnosed patients to mitigate and/or prevent the development of the symptoms. In order to do that, one of the critical pre-requisites is to have a continuous monitoring of reliable and sufficient information about users and to detect when is the right moment to intervene with a necessary check-up, or preventative strategies. The present invention presents a novel solution on how to ensure reliable (minimizing uncertainty), sufficient (complete for the required purpose), and fresh (at least minimally updated for the required purpose).

This object is fulfilled by a method with the characteristics of claim 1 and by an apparatus and non-transitory computer-readable storage medium with the features of claims 10 and 12.

Embodiments of the present invention provide, according to a first aspect, a computer implemented method for early symptom detection and preventative healthcare.

The method comprises accessing medical and/or wellness information gathered from a user, said medical and/or wellness information being gathered by different data sources (e.g. medical devices, electronic health records, mobile phones, wearable devices, biochemical and/or genetic tests, diaries, questionnaires and/or surveys); determining a risk predictive model at least using the accessed medical and/or wellness information; and determining a risk of a specific health outcome using the determined risk predictive model. Based on a result of the determined risk, the method also involves defining a decision control mechanism that calibrates said determined risk predictive model by means of: computing a set of attribute scores defining freshness, sufficiency and reliability of said accessed medical and/or wellness information, said set of attribute scores being computed using the accessed medical and/or wellness information and gathered medical and/or wellness information of similar users; providing weights to the computed set of attribute scores; computing a first score using said weights and the computed set of attribute scores; computing a second score using different variables of interest and traits of the user; and determining that new information of the user and/or a health check-up is needed based on a result of said first and second scores.

The first score relates or is associated to the need for more information of the user. The second score relates or is associated to the need for a health check-up of the user.

In an embodiment, the medical and/or wellness information, the computed set of attribute scores and the computed first and second scores are stored in at least one database.

In an embodiment, the decision control mechanism also determines a usefulness parameter for a prompt to the user by implementing a probabilistic or deterministic algorithm on the data stored in said at least one database. In some embodiments, the algorithm is a probabilistic algorithm based on a machine learning model.

In an embodiment, a first alert mechanism is further implemented by comparing the computed first score with a first threshold; comparing the usefulness parameter with a third threshold; and providing a first alert for new medical and/or wellness information if the computed first score and the usefulness parameter are higher or equal than the first and third thresholds.

In an embodiment, a second alert mechanism is also implemented. This can be done by comparing the computed second score with a second threshold; comparing the usefulness parameter with a third threshold; and providing a second alert for new health check-up if the computed second score and the usefulness parameter are higher or equal than the second and third thresholds.

The first and second alerts can be provided to a computing device such as a mobile phone, a computer, a smartwatch, a tablet, etc. of the user or of another user relative to the user.

The weights can be equal for each of the set of attribute scores, or can be provided based on a level of priority of the set of attribute scores, or can be even learned by a machine learning model from medical and/or wellness information coming from similar users.

Embodiments of the present invention also provide, according to a second aspect, an apparatus for early symptom detection and preventative healthcare. The apparatus comprises one or more processors and a non-transitory computer-readable storage medium comprising instructions, that when executed, control the one or more processors to be configured for accessing medical and/or wellness information gathered from a user, said medical and/or wellness information being gathered by different data sources; determining a risk predictive model at least using the accessed medical and/or wellness information; determining a risk of a specific health outcome using the determined risk predictive model; and defining a decision control mechanism that calibrates said determined risk predictive model by means of computing a set of attribute scores defining freshness, sufficiency and reliability of said accessed medical and/or wellness information, said set of attribute scores being computed using the accessed medical and/or wellness information and gathered medical and/or wellness information of similar users; providing weights to the computed set of attribute scores; computing a first score using said weights and the computed set of attribute scores; computing a second score using different variables of interest and traits of the user; and determining that new information of the user and/or a health check-up is needed based on a result of said first and second scores.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

The present invention thus provides an end-to-end solution that dynamically optimizes information gathering to each individual to enable personalized symptom tracking, prevention and therapy planning. The present invention is complementary with the existing Prior-Art in the following manner:

- Data collection: previous inventions cover a wide range of technical problems that are important to ensure reliable data sources, data security, transfer, privacy preserving, data provenance, etc. - assuming a safe and secure data transfer and the ensured data sources, the present invention ensures completeness, freshness, and certainty for the defined use-case. For example, the data can be provided from a reliable source and transferred in a privacy preserving way but it may not be "fresh enough" for the defined use-case.

- Treatment: a key to the present invention is the loop of the therapy planning and the frequency ("freshness"), sufficiency ("completeness") and reliability ("uncertainty") of information needed for this use case. For example, although the protocol typically defines 1 mammogram per year for 45-54 year old women, if the risk is perceived higher at an individual level for a specific person having 1 year old mammogram may be outdated. In practice, overwriting of protocol is done by doctors at an individual level when having check-ups. The idea behind this invention is to enable technology that, given the right information, can do this automatically. The present invention builds on the vast amount of inventions that defined how technologies can control for and deliver the existing medical protocols, and define the therapy, with an important difference that the loop between ensuring the appropriate data and the symptom tracking/therapy protocols to each individual.

- Adherence: providing appropriate nudge and mechanisms for incentivizing adherence were defined in previous applications, research papers and inventions. The present invention defines "what" is important to adhere to, and it is complementary to the prior art that defines how to help adherence. Very often defining which data is important and which data is not important (or which therapy is preventative and which is not) can optimize the adherence guidelines making it easier to adhere to the prescribed therapy.

- Alerts: In the present invention, providing timely alerts is an additional advantage of the mentioned loop between data gathering and the symptom monitoring & therapy planning. Specificity of present invention's approach is that it does not only focus on detecting important events but it also considers which data is critical to provide an alarm and therefore to signalize when there is a risk of inappropriate alarming (e.g. once a specific data with high predictive power is not coming in an appropriate form).

Brief Description of the Drawings

The previous and other advantages and features will be more fully understood from the following detailed description of embodiments, with reference to the attached figures, which must be considered in an illustrative and non-limiting manner, in which:

Fig. 1 schematically illustrates the different modules/elements used by the proposed method for early symptom detection and preventative healthcare, according to an embodiment of the present invention.

Fig. 2 schematically illustrates another embodiment of the different modules/elements used by the proposed method for determining the risk predictive model.

Fig. 3 illustrates an embodiment of the different sensors/data sources that can be used for gathering the medical and/or wellness information of a user(s).

Fig. 4 schematically illustrates another embodiment of the invention.

Fig. 5 schematically illustrates the balancing of the data requirements for achieving defined levels of accuracy and confidence, according to embodiment of the present invention.

Fig. 6 schematically illustrates another embodiment of the invention.

Fig. 7 schematically illustrates another embodiment of the invention.

Detailed Description of the Invention and of Preferred Embodiments

The present invention presents an end-to-end solution for delivering prompts when more data or more updated data or more reliable data 110 is needed for predicting the risk 140 of specific health outcomes. Therefore, it provides two different kinds of alerts - data alert and check-up. The data requirements (for freshness 151, completeness 152 and uncertainty/reliability 153) are individually set and dynamically updated. The former refers to different tailored requirements for each user (e.g. an older chronically ill individual at a high risk for a heart-attack would need more updated ECG information than a young healthy person). The latter refers to dynamical updates of the data requirements based on the individual state but more importantly based on a continuously updated risk prediction model 130 (e.g. with more data 110 gathered) and also in the usefulness of prompts. The system/apparatus can provide a confident prediction with less strict data requirements.
Therefore, the key aspects of this invention can be summarized as:

1) Its data-driven nature that allows for a continuous health monitoring of individuals that enables prevention intervention.
2) Personalized policies for check-ups and interventions (e.g. whereas the general recommendation for 30-40 is an annual check-up for diabetes and hypertension, for one specific person can be less or more frequent based on the captured parameters and the risk predictive model).

The present invention is adaptable for a range of data sources 100 and health domains. This can include from physiological sensing technologies and behavioral modelling from smartphone data to self-reports and parameters captured through typical diagnostic methods (e.g. doctoral observations, blood analysis, etc.).
The present invention provides the following characteristics:

- an integrated analysis of information 110 about an individual, which considers the relationship between different data inputs and in the context of other individuals and across different use-cases.
- continuous evaluation whether the existing data 110 about an individual meet the requirements for the risk prediction 140 of a specific outcome.
- a mechanism of trading-off data requirements (consisted of freshness 151, completeness 152, and uncertainty 153) for maintaining the confidence of prediction. For example, having a high certainty in one data source can offset the need for having very fresh data of the other source.
- a scalable method that assesses the risk continuously as well as the confidence 154 of the risk prediction 140, and according to this confidence it prompts for more data, or data update, or more certain data and/or prompts for a check-up (to verify the predicted risk and update the health outcome).

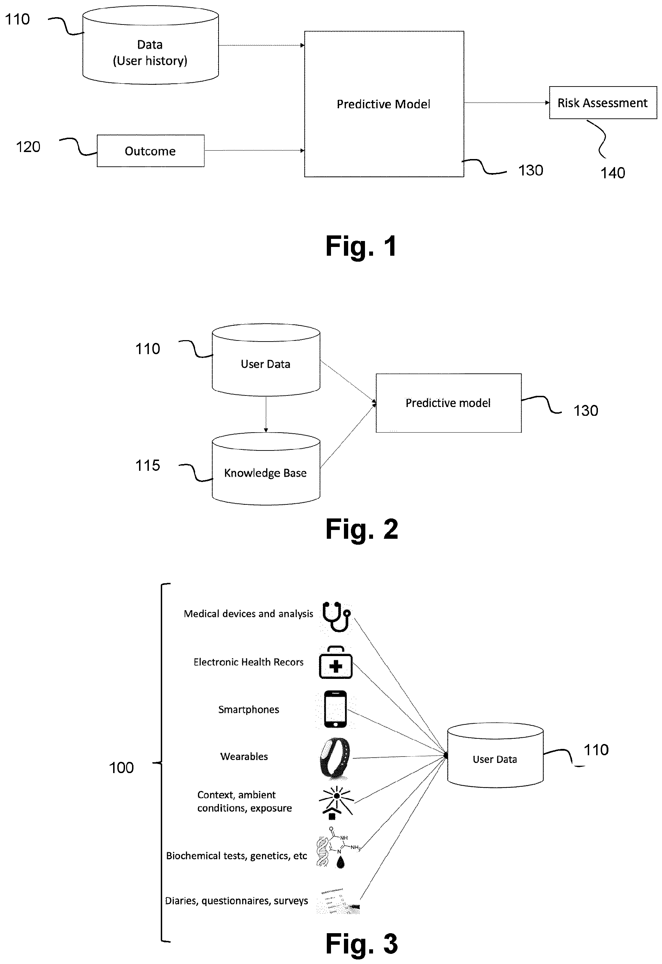

Fig. 1 schematically illustrates the main concept of the risk assessment of a specific health outcome. The present invention considers a continuous or a semi-continuous medical and/or wellness information collection 110 from different data sources 100 (see Fig. 3), which constitutes "user history" information, from the first available information about the user until the present moment. The role of the predictive model 130 is to estimate the risk 140 of outcome based on the data available to date. Conceptually, this is similar to medical guidelines built as statistics of risk factors with respect to patient's static characteristics and dynamic states (e.g. health status behaviors over time) - e.g. a person older than 50 whose family has a history of coronary problems (i.e. a genetically predisposition as a static personal characteristic) and who has a sedentary lifestyle has a higher risk of heart attack than the average person. Such statistics are made based on the typical medical history information and the related guidelines are derived. The advent of wearable devices that can measure behaviors and physiological signals, advance medical devices that are continuously improving diagnostics and assessment of a multitude of health parameters, and an increasingly adopted practice of storing more information in electronic health records, can enable personalized and continuous risk assessment at an individual level. In this way, based on aggregated data from a huge number of individuals, the models for individual risk assessment can act in a preventative way and suggest more or less frequent medical check-ups beyond the general guidelines.

Similar to the collection of statistics for patients/healthy individuals in traditional medicine, the predictive model 130 can also rely on the data from a set of a number of individuals ("knowledge base" 115) in order to assess the risk 140 of health outcomes for each individual (user data) (see Fig. 2).

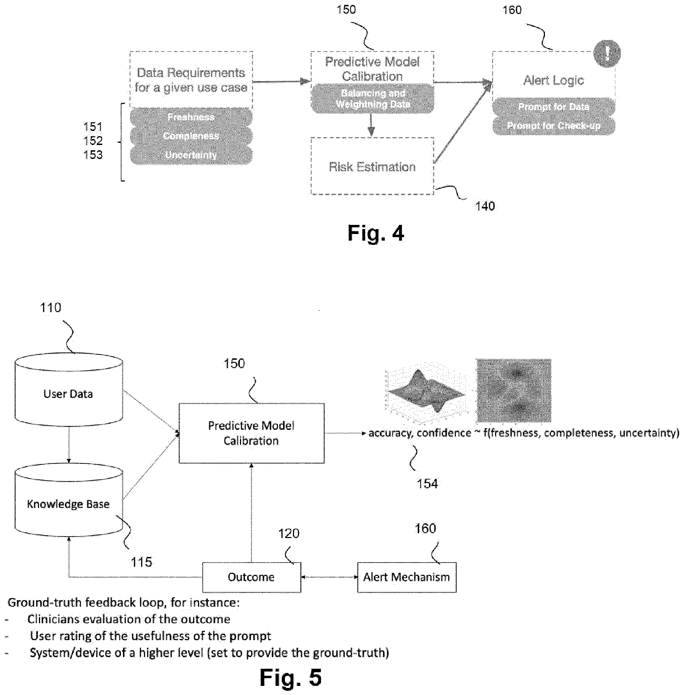

With regard to Fig. 4, therein it is illustrated an embodiment of the method for early symptom detection and preventative healthcare illustrating its key elements: data requirement attributes of freshness 151, completeness 152 and uncertainty 153; concept of balancing and weighting data requirements (freshness, completeness and uncertainty) for achieving defined levels of accuracy and confidence 154 and the corresponding calibration of the predictive model 150 (see Fig. 5); building risk estimations 140 and prevention healthcare mechanisms at an individual level; and alert mechanism 160 that prompts for a) data revision, b) check-up.

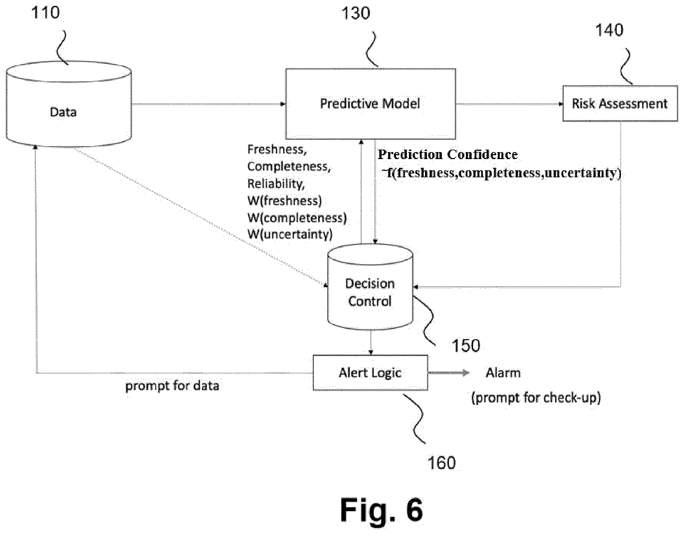

Fig. 6 illustrates another embodiment of the present invention. According to this embodiment, the data collection and/or storage module 110 is responsible for extracting dynamic information from a user as per the above descriptions that will be further used to compute the freshness 151, completeness 152 and uncertainty 153 scores, as well as by the predictive model 130. A decision control module 150 computes the freshness 151, completeness 152 and uncertainty 153 scores per user based on the data gathered 110 from the current user and also from similar individuals, both passive and active. This will inform the calibration mechanism of the decision control module 150 (that can be based on machine learning algorithms) through the prediction confidence 154 which therefore estimates how useful a fresh prompt to the user would be. Based on this, it is possible to balance among the data requirements (freshness, completeness, uncertainty as illustrated on Fig. 5). The decision control module 150 also iterates different weights (described below) assigned to freshness, completeness, uncertainty 151-153 in order to find an optimal point for the risk estimation confidence (Prediction Confidence) 154 based on the constraints of data 110 gathering. The weights can be manually overwritten based on the data constraints in order to achieve a new balance within acceptable margins for the remaining data requirements. Then this module 150 computes based on these a need for data score and a need for checkup score. Decision control module 150 has another important role - prediction of the prompt usefulness - using data describing the user's past information, a model is trained to predict how useful a future prompt will be for each user. The output obtained from this model is used to inform the alert logic module 160. This prediction is done also by taking into account previous feedback in similar contexts and use cases.

The alert logic module 160 can set the criteria when the prompt for data or prompt for checkup are raised. This module 160 is responsible to flag whether the system/apparatus needs extra data and whether the user needs a check-up at the set moment. In an embodiment, the module 160 receives as input the outputs from the decision control module 150 (i.e., the scores of freshness 151, completeness 152, uncertainty 153, their corresponding weights, as well as the prediction of the prompt usefulness). The alert logic module 160 uses the inputs from the decision control module 150 in order to decide whether to do the prompts based on the needs and expected usefulness given the use case.

Present invention can further include a feedback mechanism module in charge of assessing the data revision and/or a check-up need (such as from a user and a doctor or a high level system, etc.). The feedback gathered can be implicit, if there is no need of interaction with any of the two or explicit, whenever a follow up feedback inquiry is sent to the user or doctor. The feedback gathered is fed back to the system/apparatus to improve future risk predictions or future predictions of the data requirements attributes (Freshness, completeness, uncertainty).

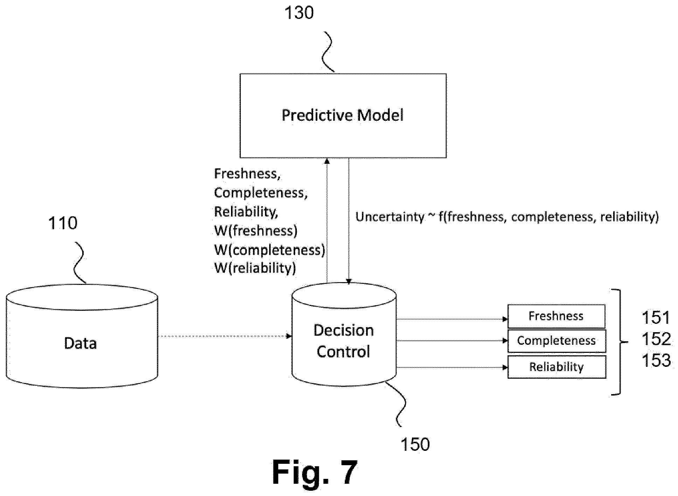

Fig. 7 illustrates another embodiment of the present invention. Data collection and/or storage module (user history) 110 is a module that has the collection of the past and an ongoing records of medical history from Electronic Health Records (EHR) and/or self-reported symptoms (e.g. pain, happiness, sleep quality, stress, fever, etc.), self-reported medical history, self-reported personal characteristics (such as personality, demographics, etc.), passively collected data from smartphones and/or wearables (e.g. heart-rate, galvanic skin response, body temperature, location, phone unlocks, light, speech, ambient noise, phone use, as well as features built on top of the sensors-data e.g., time spent at home, phone use, daily battery consumption), and/or contextual information (e.g., weather, day of the week, local holidays calendar).

Decision control module 150 is responsible for computing and verifying periodically the data requirements at user individual level for:

- Freshness 151: This module verifies how fresh the user records are and computes a score based on how recent the user added information to the system (both passive and active).
- Completeness 152: This module verifies how complete the user file is. The score represents a percentage of how complete the last information added to the system is.
- Uncertainty 153: This module verifies how uncertain the user file is. The score computed is based both on other individual's provided information in similar contexts, as well as the individual's history in similar contexts.

The decision control module 150 is also responsible for the balancing of the data requirements for achieving defined levels of accuracy and confidence 154, and for predicting the usefulness of a prompt.

Freshness:

The goal of this module is to compute and output the freshness score 151 of the stored data 110 of the user(s) of interest depending on the use case and the relevant Outcome 120 ground truth target variable (e.g., wellbeing). The freshness score 151 captures the notion of how updated the information in the system is based on the requirements of the use case (e.g., daily records of time spent at home for depressive patients). Moreover, depending on how static and variable in general the variable is, different requirements can be set. For example, if some data remains constant for a long period, this data will be fresh for a longer time, e.g., gender, while if it is highly varying, it might need more frequent updates, e.g., body temperature.

A parameter called "Age" of information is defined to quantify the freshness score 151. For a given data component d, a freshness metric is defined in terms of the last timestamp in which the data has been collected, $t_{last,d}$, and the current time , $t_{now}$ with the utility function that depends on both values. For example:

$$u_d\left(t_{last,d}, t_{now}\right) = max(0, 1 - m_d \times (t_{now} - t_{last,d})) \qquad for \quad m_d = 1/t_{max,d}$$

where $m_d$ quantifies how fast the utility of the data diminishes over time and tmax,d is the maximum time after which the data is fresh, i.e., the utility is 0. Many other utility functions can be considered, e.g., exponential or polynomial.

The freshness score 151 can be provided by data or as the average of all the individual data. Importantly the function parameters can be continuously updated.

For instance, for a person suffering from high blood pressure, the measurements related to blood pressure are expected to be weekly, leading to md = 1/7 = 0.14. In the case that it has been 10 days with the person not providing this data, the utility function would retrieve a freshness score of: max(0, 1 - 0.14*(10-0)) = max (0, -0.4) = 0, where tnow=10 and tlast =0 . Thus the utility as given by the age of parameter would be 0, denoting how fresh the measurement of blood pressure of this person is (the lower, the less fresh the information is).

Completeness:

The goal of this module is to compute the completeness score 152 of the stored user's data 110 based on the individual's use case. The completeness score 152 captures the notion of how complete the information in the system is based on how which data is required for a specific outcome prediction. For example, the body temperature and resting state heart rate might be enough to predict the upcoming flu but only temperature would not be sufficient.

A possible embodiment of completeness is a boolean vector for the available data required for the specific task for each of the data components.

Uncertainty:

The data is not always fully accurate - e.g. temperature measurement suffer from the sensitivity of a device, self-reports prone to subjectivity and recall bias, behavioral features captured from the mobile phone might be affected by the sensor limitations, battery level that can impact sampling rate for different sensors which can also stop sending the data. The goal of this module is to compute uncertainty scores 153 given the use case requirements and relevant data both from the user's past data as well as data from similar individuals.

This score 153 can be interpreted as how unrepresentative the current user information is compared to historical individual's data as well as to the data from similar individuals, in particular referring to the certainty that the capture information is enough for predicting the outcome (i.e. the associated risk 140).

One example of the system inferring a low uncertainty score 153 of the captured information is a self-reported subjective wellbeing score for an extroverted individual who spent a considerably higher amount of time at home than usual, was not in touch with his friends/family as compared with the past records. As an example, the system would have searched for self-reported subjective wellbeing scores of the other individuals in similar contexts with similar personality traits - based on the quantified discrepancy with typically reported subjective wellbeing scores the system would compute the uncertainty score (in this example a low uncertainty score).

The interpretation of this score 153 can be twofold: (a) the measurements captured through the sensors 100 and active data are incorrect due to faulty circumstances (e.g., forgetting the phone on the desk/at home all day, leaving the phone with the kid, etc.). In this case, the uncertainty score 153 would be low; (b) the measurements captured through the sensors 100 and active data are correct, and the user might be experiencing an out of the ordinary situation that is impacting his wellbeing. In this case, the uncertainty score 153 would be high.

As previously indicated, the present invention can further balance the data requirements for achieving defined levels of accuracy and confidence 154. To do so, dynamic information extracted for the user (freshness, completeness and uncertainty 151-153 and the variables of interest) is used to compute a need for data score (ND score or first score as termed in the claims) and a need for check-up score (NC score or second score as termed in the claims) based on the considered use case. Those scores will be taken into account in the Alert Logic module 160 to decide whether a prompt for data or a prompt for checkup is activated.

The ND score can be computed as:

$$ND = w_0 * freshness + w_1 * completeness + w_2 * uncertainty;$$

where, $w_0$, $w_1$ and, $w_2$ and $w_3$ are weights that emphasise the value each input variable receives and ($\sum w_i = 1$). In one embodiment, the weights can be equally proportionate, i.e., 0.33 each. In another embodiment, the clinician/client can set the weights themselves based on their priority and use case. In another embodiment, the weights can be learnt by a machine learning model from data coming from other individuals in a similar use case to understand which of the components is more relevant in general and for that specific moment considering the variables of interest and the rest of the data captured. This calibration of the data requirements is a crucial component for balancing across different data categories depending on the available data, its completeness and uncertainty scores. For instance, let's consider an individual that currently requires its blood pressure, heart rate and breathing rate to be monitored on a weekly basis due to a recent change in its medication. Given a 3 weeks of missing measurements in breathing rate, but fresh and complete measurements for the other two categories, the alert logic 160 may decide not to raise a prompt for data, if the other two measurements are sufficient for reaching the desired level of accuracy for the use case.

The NC score can be computed as a function of the variables of interest and the traits of the user, and outputs as a result $NC \in \{none, low, medium, high\}$. In embodiment, there is only one variable of interest and the clinician sets the three necessary thresholds (between none and low, low and medium, medium and high) on the value of this variable depending on the user's traits. In another embodiment, the thresholds are dependent only on the variable of interest, independently of the user's traits and can be decided by the system or by the doctor. In another embodiment with multiple variables of interest $vi$, each variable is treated independently to get a $NC_{vi}$ score, those scores would be converted to the values 0, 1, 2, 3 and added with a weight of importance: $NC = \Sigma_{vi} w_{vi} \cdot NC_{vi}$, with $\Sigma_{vi} w_{vi} = 1$. The result is rounded and mapped back to $\{none, low, medium, high\}$.

In some embodiments, the usefulness of data revision based on the past records can be also predicted. It can be probabilistic (e.g. a machine learning model) or deterministic. After each data revision, ground-truth information about the outcome (in case of check-up) or usefulness of the new data for the model (in case of data prompt) is gathered. The machine learning model can use various input variables including (but not limited to) usefulness ratings of past prompts, psychometric traits (e.g., big five personality, sense of control etc.), past behaviors (e.g., relevant activities, visits to the clinician, actions taken upon presentation of a prompt etc.), freshness, completeness and uncertainty given the use case. By using a machine learning regression algorithm, the output of a machine learning model for a user $user_i$ is a score, $pred\_u_{user_i}$ that can vary between 1 to 10 based on the scale used for rating the usefulness.

In some embodiments, the machine learning model can leverage XGBoost, an ensemble tree-based learning method that is well known for being robust with missing data, and thus complements very well the calibration component.

The alert logic module 160 particularly uses the ND score, the NC score and the predicted usefulness score in order to decide whether a prompt for data or a prompt for check-up is activated. The prompt can be sent to a user, an informal or formal caregiver, medical staff, entity, person, other system, device or similar which is allowed to define "ground-truth" about the outcome and then inform the Feedback mechanism component.

If the ND score is above a certain threshold $T_{ND}$ as well as the predicted usefulness score is higher than a certain threshold $T_{pred\_u_{user_i}}$, the system requires new data to be acquired. The predicted usefulness score is then used to determine whether the user must be prompted directly to take action or the prompt should go to another person from the user's circle. It may be the case that the data that is missing can only be gathered through a visit to the clinician. In that case, the user would need to go to the clinician to gather that data and input it to the system, following this prompt.

If the NC score is above a certain threshold $T_{NC}$ as well as the predicted usefulness score is higher than a certain threshold $T_{pred\_u_{user_i}}$, the system prompts for a check-up. Moreover, in one embodiment, check-up can also mean gather new information about the user, about the outcome, or risk factor - therefore it can also be triggered when an uncertainty score 153 that is high, or the freshness 151 or completeness 152 score being low, and when the predicted usefulness score are high enough.

This type of alert targets the user for early detection of symptoms and asks for additional information in case the system detects that the user file is either: deprecated/stale, incomplete, or the uncertainty level detected by the system is high.

The prompt score is general and is used for early detection of symptoms, timely provision of prompts and deciding when new data must be collected. The prompt score can be further grouped into discrete states such a low, medium and high to indicate the severity of the condition, and this state indicates what action the user or clinician will be asked to take. The prompt score can also be binarized - i.e., given a value of 1 or 0 (if the score is above or below a threshold, respectively) to decide when historical information about the user needs to be refreshed.

In an embodiment related to mental health, the prompts can be sent to friends/family of the user instead of the user. The user can define a 'friends and family' circle that consists of all the people in a user's life that are important to them. The user is always in the center of the circle (first level), followed by their closest ally (e.g., partner) in the second level, and followed by their best friend in the third level and so on. The clinician and a close neighbor can be placed in the third or fourth level, based on the user's past behavior. A system of prompts can be built where for levels of low severity, only the user is prompted to take action. For medium severity conditions, the user and their closest ally in the second level are prompted on their respective mobile phones. Thus, there is a follow up done in the physical work that compels the user to take action. In cases of high severity, all members of the circle within the third or fourth levels are informed, so that there is a follow up from all of the user's trusted allies in the physical world to push the user to visit a clinician or do a checkup.

The feedback mechanism is in charge of assessing the data revision and/or a check-up need (such as from a user and a doctor or a high level system, etc.). The feedback can be reported by a user, and/or a clinician and/or an entity, person, the other device or a system considered as "ground-truth", which defines the utility of the data/check-up prompt. In particular, the feedback gathered can be implicit, if there is no need of interaction with any of the two or explicit, whenever a follow up feedback inquiry is sent to the user or doctor. In an embodiment the usefulness of a prompt can be learnt automatically by the system based on the actions of the user (e.g., providing fresh updated data in the case of a prompt for data, or attending a checkup in the case of a prompt for checkup).

Furthermore, the feedback gathered is fed back to the system to improve future risk predictions or future predictions of the data requirements attributes (Freshness 151, completeness 152, uncertainty 153). In particular, the feedback is then taken into consideration by the decision control module 150 for calibrating the model 130 (for instance adjusting the weights for balancing the data requirements based on observed important data for the prompt.

Following a particular example of the uncertainty score 153 computation is detailed.

**Case I:** Given the ground truth, i.e., an active current wellbeing momentary measurement for an individual i, past records belonging to the current individual and individuals with similar traits with a similar active wellbeing measurement are filtered, and the following uncertainty score 153 is computed:

$$Uncertainty^i(t) = 1/n * \sum (S^i_f(t_{present}) - 1/m * \sum S^{i+s^i}_f(t_{past})|)$$

The score computes the average absolute difference for each sensor feature $S^i_f$ with f from 1 to n, between the current sensor feature measurement $S^i_f(t_{present})$ and the mean of past sensor feature measurements $S^{i+s^i}_f(t_{past})$, both from this individual *i* and individuals with similar traits $s^i$, with *i* from 1 to m, when reporting a similar active wellbeing measurement.

In this particular case, the uncertainty score 153 corresponds to interpretation (a) above.

For example, let's consider the light level and noise level as sensor-based feature, and the following records:

| Individual | Light level [0-1] | Noise level [0-1] | Wellbeing reported [0-1] |
|---|---|---|---|
| i | 0.1 | 0.7 | 0.7 |
| i | 0.7 | 0.2 | 0.7 |
| $s^1$ | 0.8 | 0.1 | 0.7 |
| $s^2$ | 0.9 | 0.3 | 0.7 |
| $s^3$ | 0.7 | 0.2 | 0.7 |

In this case, the module searches for records with similar wellbeing reported. This leads to filtering records of the individual i and similar individuals with similar reported wellbeing, producing the table 1 above.

The uncertainty score 153 would be: $1/2 * [|0.1 - (0.7 + 0.8 + 0.9 + 0.7) * ¼|] + [|0.7-(0.2 + 0.1 + 0.3 + 0.2) * ¼|] = ½ * [0.675 + 0.5] = 0.59$.

This corresponds to a high uncertainty score 153, meaning that most likely the measurements from the sensors were faulty.

**Case II:** When the ground truth information is missing, the model searches for records of individuals with similar traits and in similar contexts and matching passive sensor measurements, computes their mean wellbeing target score, and based on that retrieves records of the current individual from the past where the user reported similar wellbeing scores. Based on these records, it then computes the uncertainty score 153 based on the formula above.

In the majority of the cases the uncertainty score 153 in this case would be low, based on the assumption that there is a vast literature on predicting wellbeing scores from passive sensing data.

In this particular case, the uncertainty score 153 corresponds to interpretation (b) above.

For example, let's consider the light level and noise level as sensor-based feature, and the following records:

| Individual | Light level [0-1] | Noise level [0-1] | Wellbeing reported [0-1] |
|---|---|---|---|
| i | 0.1 | 0.7 | NA |
| i | 0.3 | 0.9 | 0.3 |
| $s^1$ | 0.2 | 0.6 | 0.3 |
| $s^2$ | 0.3 | 0.7 | 0.3 |

The first row represents the current row under analysis.

The first step involves retrieving records with similar sensors measurements for the sensors recorded of the same individual i and similar individuals, resulting in rows 2-4. The second step involves computing the average wellbeing in the records, resulting in wellbeing of 0.3. The third step involves filtering records of the individual i of similar wellbeing, finally producing the table above.

The uncertainty score 153 would be: $1/2 * [|0.1 - (0.3 + 0.2 + 0.3) * ⅓|] * [|0.7 - (0.9 + 0.6 + 0.7) * ⅓|] = ½ * [0.16 + 0.03] = 0.095$

This corresponds to a low uncertainty score 153, meaning that indeed the user might be exhibiting an unusual behavior.

Various aspects of the proposed method, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at anytime for the software programming.

All or portions of the software may at times be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with data processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

A machine-readable medium may take many forms, including but not limited to, a tangible storage medium, a carrier wave medium or physical transmission medium. Non-volatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data.

Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described herein may be embodied in a hardware device, it may also be implemented as a software only solution-e.g., an installation on an existing server. In addition, data processing as disclosed herein may be implemented as a firmware, firmware/software combination, firmware/hardware combination, or a hardware/firmware/software combination.

The present disclosure and/or some other examples have been described in the above. According to descriptions above, various alterations may be achieved. The topic of the present disclosure may be achieved in various forms and embodiments, and the present disclosure may be further used in a variety of application programs. All applications, modifications and alterations required to be protected in the claims may be within the protection scope of the present disclosure.

The scope of the present invention is defined in the following set of claims.

**Claims**

1. A computer implemented method for early symptom detection and preventative healthcare, comprising:

   - accessing, by a processor, medical and/or wellness information (110) gathered from a user, said medical and/or wellness information (110) being gathered by different data sources (100);
   - determining, by the processor, a risk predictive model (130) at least using the accessed medical and/or wellness information (110);
   - determining, by the processor, a risk (140) of a specific health outcome using the determined risk predictive model (130); and
   - defining, by the processor, a decision control mechanism (150) that calibrates said determined risk predictive model (130) by means of:

      - computing a set of attribute scores (151, 152, 153) defining freshness, sufficiency and reliability of said accessed medical and/or wellness information (110), said set of attribute scores (151, 152, 153) being computed using the accessed medical and/or wellness information (110) and gathered medical and/or wellness information of similar users;
      - providing weights to the computed set of attribute scores (151, 152, 153);
      - computing a first score using said weights and the computed set of attribute scores (151, 152, 153), the first score relating to a need for more information of the user;
      - computing a second score using different variables of interest and traits of the user, the second score relating to a need for a health check-up of the user; and
      - determining that new information of the user and/or a health check-up is needed based on a result of said first and second scores.

2. The method of claim 1, wherein the medical and/or wellness information (110), the computed set of attribute scores (151, 152, 153) and the computed first and second scores are stored in at least one database.

3. The method of claim 2, wherein the decision control mechanism further comprises determining a usefulness parameter for a prompt to the user by implementing a probabilistic or deterministic algorithm on the data stored in said at least one database.

4. The method of claim 3, wherein said algorithm is a probabilistic algorithm based on a machine learning model.

5. The method of claim 3 or 4, further comprising implementing a first alert mechanism that:

   compares the computed first score with a first threshold;
   compares the usefulness parameter with a third threshold; and
   provides a first alert for new medical and/or wellness information if the computed first score and the usefulness parameter are higher than the first and third thresholds.

**6.** The method of claim 3, 4 or 5, further comprising implementing a second alert mechanism that:

> compares the computed second score with a second threshold;
> compares the usefulness parameter with a third threshold; and
> provides a second alert for new health check-up if the computed second score and the usefulness parameter are higher than the second and third thresholds.

**7.** The method of claim 6, wherein the first and second alerts are provided to a computing device of the user or of another user relative to the user.

**8.** The method of any one of previous claims, wherein the weights are equal for each of the set of attribute scores (151, 152, 153), or wherein the weights are provided based on a level of priority of the set of attribute scores (151, 152, 153), or wherein the weights are learned by a machine learning model from medical and/or wellness information coming from similar users.

**9.** The method of any one of previous claims, wherein the different data sources (100) comprises medical devices, electronic health records, mobile phones, wearable devices, biochemical and/or genetic tests, and diaries, questionnaires and/or surveys.

**10.** An apparatus for early symptom detection and preventative healthcare, comprising:

> at least one processor;
> a non-transitory computer-readable storage medium comprising instructions, that when executed, control the at least one processor to be configured for:
>
>> - accessing medical and/or wellness information (110) gathered from a user, said medical and/or wellness information (110) being gathered by different data sources (100);
>> - determining a risk predictive model (130) at least using the accessed medical and/or wellness information (110);
>> - determining a risk (140) of a specific health outcome using the determined risk predictive model (130); and
>> - defining a decision control mechanism (150) that calibrates said determined risk predictive model (130) by means of:
>>
>>> - computing a set of attribute scores (151, 152, 153) defining freshness, sufficiency and reliability of said accessed medical and/or wellness information (110), said set of attribute scores (151, 152, 153) being computed using the accessed medical and/or wellness information (110) and gathered medical and/or wellness information of similar users;
>>> - providing weights to the computed set of attribute scores (151, 152, 153);
>>> - computing a first score using said weights and the computed set of attribute scores (151, 152, 153), the first score relating to a need for more information of the user;
>>> - computing a second score using different variables of interest and traits of the user, the second score relating to a need for a health check-up of the user; and
>>> - determining that new information of the user and/or a health check-up is needed based on a result of said first and second scores.

**11.** The apparatus of claim 10, further comprising a database configured to store the medical and/or wellness information (110), the computed set of attribute scores (151, 152, 153) and the computed first and second scores.

**12.** A non-transitory computer-readable storage medium containing instructions, that when executed, control a computer system to be configured for:

> - accessing medical and/or wellness information (110) gathered from a user, said medical and/or wellness information (110) being gathered by different data sources (100);
> - determining a risk predictive model (130) at least using the accessed medical and/or wellness information (110);
> - determining a risk (140) of a specific health outcome using the determined risk predictive model (130); and
> - defining a decision control mechanism (150) that calibrates said determined risk predictive model (130) by means of:

- computing a set of attribute scores (151, 152, 153) defining freshness, sufficiency and reliability of said accessed medical and/or wellness information (110), said set of attribute scores (151, 152, 153) being computed using the accessed medical and/or wellness information (110) and gathered medical and/or wellness information of similar users;
- providing weights to the computed set of attribute scores (151, 152, 153);
- computing a first score using said weights and the computed set of attribute scores (151, 152, 153), the first score relating to a need for more information of the user;
- computing a second score using different variables of interest and traits of the user, the second score relating to a need for a health check-up of the user; and
- determining that new information of the user and/or a health check-up is needed based on a result of said first and second scores.

13. The non-transitory computer readable medium of claim 12, wherein the instructions are further configured to store the medical and/or wellness information (110), the computed set of attribute scores (151, 152, 153) and the computed first and second scores in at least one database.

14. The non-transitory computer-readable medium of claim 13, wherein the instructions are further configured to determine a usefulness parameter for a prompt to the user by implementing a probabilistic or deterministic algorithm on the data stored in the database.

15. The non-transitory computer-readable medium of claim 14, wherein the algorithm is a probabilistic algorithm based on a machine learning model.

**Fig. 1**

**Fig. 2**

**Fig. 3**

150 160

Data Requirements
for a given use case

151
152
153

Freshness
Completeness
Uncertainty

Predictive Model
Calibration

Balancing and
Weighting Data

Risk Estimation

140

Alert Logic

Prompt for Data
Prompt for Check-up

## Fig. 4

110

User Data

150

Predictive Model
Calibration

accuracy, confidence ~ f(freshness, completeness, uncertainty)

154

Knowledge Base

115

120

Outcome

160

Alert Mechanism

Ground-truth feedback loop, for instance:
- Clinicians evaluation of the outcome
- User rating of the usefulness of the prompt
- System/device of a higher level (set to provide the ground-truth)

## Fig. 5

110 130 140

Data → Predictive Model → Risk Assessment

Freshness,
Completeness,
Reliability,
W(freshness)
W(completeness)
W(uncertainty)

Prediction Confidence
~f(freshness,completeness,uncertainty)

Decision
Control

150

Alert Logic → Alarm
(prompt for check-up)

prompt for data

160

# Fig. 6

**Fig. 7**

Europäisches
Patentamt

European
Patent Office

Office européen
des brevets

# EUROPEAN SEARCH REPORT

Application Number

EP 20 38 2572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Erin P Balogh ET AL: "Improving Diagnosis in Health Care" In: "Improving Diagnosis in Health Care", 29 December 2015 (2015-12-29), National Academies Press, Washington, D.C., XP055724936, ISBN: 978-0-309-37769-0 * page 32 last par, page 48 last full par, page 49 first full par and last 2 paragraphs, page 50 first par, page 51 first full par * | 1-15 | INV. G16H50/20 G16H50/70 |
| A | Anonymous: "Artificial intelligence in healthcare - Wikipedia", , 13 June 2020 (2020-06-13), XP055724943, Retrieved from the Internet: URL:https://en.wikipedia.org/w/index.php?title=Artificial_intelligence_in_healthcare&oldid=962354941 [retrieved on 2020-08-25] * page 1 par 1 * | 1-15 | |
| A | CN 110 021 434 A (YIMAO TECH DEVELOPMENT SHANGHAI CO LTD) 16 July 2019 (2019-07-16) * par 127 * | 1-15 | TECHNICAL FIELDS SEARCHED (IPC) G16H |

The present search report has been drawn up for all claims

| Place of search | Date of completion of the search | Examiner |
|---|---|---|
| Munich | 19 November 2020 | Bankwitz, Robert |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

 

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 20 38 2572

This annex lists the patent family members relating to the patent documents cited in  the above-mentioned European search report.
The members are as contained in the European Patent Office EDP file on
The European Patent Office is in no way liable for these particulars which are merely given for the purpose of information.

19-11-2020

| Patent document cited in search report | Publication date | Patent family member(s) | Publication date |
|---|---|---|---|
| CN 110021434 A | 16-07-2019 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110225007 A1
- US 7395215 B2
- US 7540841 B2
- CN 101526980 A
- US 20130226617 A1
- US 8566121 B2
- US 20170098050 A1
- US 20060058612 A1
- US 5576952 A
- US 20080214944 A1
- US 20110245633 A1